# EUROPÄISCHE PATENTANMELDUNG

(11) **EP 2 695 576 A1**
(43) Veröffentlichungstag der Anmeldung: **12.02.2014**
(21) Anmeldenummer: 13165967.4
(22) Anmeldetag: 30.04.2013
(51) Int. Cl.: A61B 5/05, A61N 1/40, A61N 1/04, A61B 5/053

(54) **Elektrodenendgerät für die Bioresonanztherapie und -diagnostik**

(30) Priorität: 07.08.2012 DE 102012015572
(71) Anmelder: Von Buenger, Peter, 82054 Sauerlach-Altkirchen (DE)
(72) Erfinder: Von Buenger, Peter, 82054 Sauerlach-Altkirchen (DE)
(74) Vertreter: Schwabe - Sandmair - Marx

(57) **Zusammenfassung**

Die Erfindung betrifft ein Elektrodenendgerät für die Diagnose und Therapie nach dem Prinzip des biofunktionellen Ansatzes, insbesondere für die Bioresonanztherapie, sowie ein Gerät für die biofünktionelle Diagnose und Therapie umfassend mindestens ein erfindungsgemäßes Elektrodenendgerät.

Das Elektrodenendgerät der vorliegenden Erfindung zeichnet sich durch zwei voneinander elektrisch isolierte Elektroden aus, zwischen denen eine Wechselspannung angelegt werden kann bzw. dadurch, dass das elektrisch leitende Elektrodenmaterial des Elektrodenendgerätes wenigstens teilweise mit einem elektrisch nichtleitenden Überzug versehen ist.

## Beschreibung

Die Erfindung betrifft ein Elektrodenendgerät für die Diagnose und Therapie nach dem Prinzip des biofunktionellen Ansatzes, insbesondere für die Bioresonanztherapie, sowie ein Gerät für die biofunktionelle Diagnose und Therapie umfassend mindestens ein erfindungsgemäßes Elektrodenendgerät.

Unter dem biofunktionellen Ansatz in der Diagnose und Therapie versteht man die Detektion und gezielte Beeinflussung körperspezifischer elektromagnetischer Signale, die an der Hautoberfläche des Patienten gemessen werden können. Unter den Methoden, die sich des biofunktionellen Ansatzes bedienen, fallen insbesondere die Bioresonanztherapie, die Radionik, die Elektroakupunktur, Biokommunikations-, Bicom-, Multicom- und Multiresonanztherapie, Biophysikalische Informationstherapie (BIT), Mora-Therapie, diagnostische Resonanztherapie (DRT), Sequentielle Frequenzdiagnostik, Lykotronik-Therapie, SomaDyne, vegaSTT oder Matrix-Regenerationstherapie oder auch die Informationsfeld-Medizin.

Die genannten körperspezifischen elektromagnetischen Signale treten als elektromagnetische Schwingungen (Frequenzen) in Erscheinung. Diese körpereigenen Signale und Zellpotentiale liegen im Microampere-Bereich (µA). Ähnlich den Methoden EKG sowie EEG oder des MEG, das Magnetfelder im Gehirn über an der Kopfhaut angebrachte Elektroden misst, werden nach dem Prinzip der biofunktionellen Methode die genannten körpereigenen Wechselspannungen und Ströme auf der Haut von geeigneten Elektrodenendgeräten abgegriffen. Die Frequenzen werden in einem elektrisch leitend verbundenen Zentralgerät, das auch als Schwingungsgeber, Therapiegerät, Bioresonanzgerät oder Steuergerät bezeichnet wird, analysiert und durch korrigierende therapeutische vom Zentralgerät ausgesandte elektromagnetische Schwingungen über die Elektrodenendgeräte (auch Therapieelektroden genannt) wieder an die Haut des Patienten abgegeben. Auf diese Weise wird nach der Theorie des biofunktionellen Ansatzes beispielsweise ein pathologisches Frequenzmuster, das von einem pathologischen Zustand des Organismus herrührt, korrigierend und therapeutisch beeinflusst oder im Idealfall der gesunde Normalzustand wiederhergestellt. Die Beeinflussung kann hierbei durch feststehende, vorprogrammierte Software erfolgen oder auch dadurch, dass die gemessenen körpereigenen Signale weiterverarbeitet werden und in einer Feedback-Schleife an den Patienten zurückgegeben werden.

Sowohl Elektrodenendgeräte, als auch Zentralgeräte sind im Prinzip lange bekannt. Beispiele für Zentralgeräte werden in den Patentschriften EP 1 569 938 B1, DE 195 47 309 A1, oder DE 198 31 785 A1 beschrieben.

Die Elektrodenendgeräte sind in der Regel stabförmig, können jedoch prinzipiell in jeder Form auftreten. DE 20 2008 015 761 U1 beschreibt beispielsweise flexible Textilerzeugnisse, die als Elektrodenendgeräte für die Bioresonanztherapie empfohlen werden. Allen konventionellen Elektrodenendgeräten jedoch ist gemeinsam, dass ein Elektrodenendgerät genau einen Pol verwendet. Spannungen und Ströme und die von ihnen übertragenen Signale bestehen daher zwischen mindestens zwei mit einpoligen Elektrodenendgeräten belegten Hautpartien des Patienten. Üblicherweise hält der Patient in jeder Hand ein mit elektrisch leitender Metall-oder Metallegierungsoberfläche versehenes einpoliges Elektrodenendgerät, so dass Spannung und Strom zwischen den beiden Händen des Patienten erzeugt werden und die Signale den Widerstand beider Arme und des Rumpfes überwinden können müssen, was zu hohen Strombelastungen des Patienten führen kann, die weit oberhalb, d.h. praktisch bis zu einem vieltausendfachen der körpereigenen Ströme und physiologischen Spannungen liegen. Auch wenn in Deutschland Ströme von bis zu 100 mA zulässig sind, ist eine übermäßige Belastung und eventuelle Schädigung des Organismus durch externe elektromagnetische Beeinflussung unbedingt zu vermeiden.

Die Erfindung stellt sich demnach die Aufgabe, eine besonders schonende Durchführung von Diagnostik und Therapie nach dem biofunktionellen Ansatz, insbesondere der Bioresonanztherapie zu ermöglichen, indem elektrische Ströme und Spannungen verwendet werden können, die sehr nahe am physiologischen Bereich liegen.

Dies wird erfindungsgemäß durch zwei Maßnahmen erreicht.

Zum einen betrifft die Erfindung daher ein einstückiges Elektrodenendgerät für Diagnose und/oder Therapie nach dem Prinzip des biofunktionellen Ansatzes, **dadurch gekennzeichnet, dass** es zwei voneinander elektrisch isolierte Elektroden (Pole) enthält, zwischen denen eine regelbare Wechselspannung angelegt werden kann.

Nach dem biofunktionellen Ansatz wird im allgemeinen eine beispielsweise durch das Zentralgerät regelbare Wechselspannung verwendet, die insbesondere eine dem Verlauf und der Form nach festgelegte oder gesteuerte Wechselspannung sein kann.

Zum andern betrifft die Erfindung ein einstückiges Elektrodenendgerät für Diagnose und/oder Therapie nach dem Prinzip des biofunktionellen Ansatzes, **dadurch gekennzeichnet, dass** das elektrisch leitende Elektrodenmaterial des Elektrodenendgerätes wenigstens teilweise mit einem elektrisch nichtleitenden Überzug versehen ist.

Im Idealfall vereint die Erfindung beide Aspekte, das heißt, dass das elektrisch leitende Elektrodenmaterial des Elektrodenendgerätes wenigstens teilweise mit einem elektrisch nichtleitenden Überzug versehen ist und zwei voneinander elektrisch isolierte Elektroden enthält, zwischen denen eine regelbare Wechselspannung angelegt werden kann.

"Einstückig" im Sinne der Erfindung bedeutet, dass das Elektrodenendgerät zusammenhängend ist, ohne äußere Kraftanwendung Formstabilität aufweist und mit einer Hand gehalten werden kann. Das heißt, mehrteilige Elektrodenendgeräte, die beispielsweise zusammengesteckt dieses Kriterium erfüllen, werden als "einstückig" im Sinne der Erfindung angesehen. Ebenso gelten flexible Elektrodenendgeräte, die beispielsweise durch geringen Kraftaufwand gebogen werden und somit an Körperformen optimal angepasst werden können, als "einstückig".

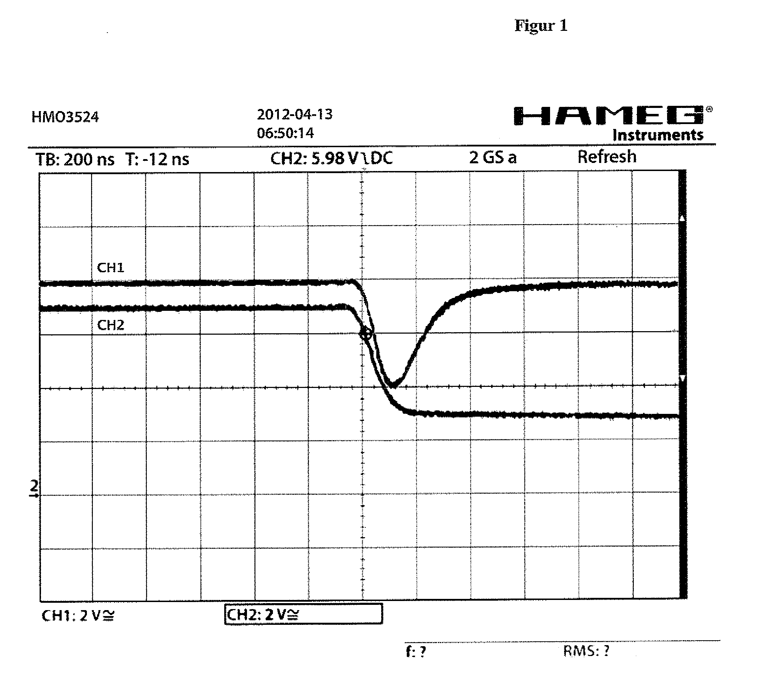

Werden erfindungsgemäß zweipolige Elektrodenendgeräte verwendet, so werden Diagnose und Therapie in derselben Elektrode bewirkt. Der Strom, der im Bedarfsfall durch die Hand fließt - wenn die Elektrodenendgeräte in einer Hand gehalten werden - kann kleiner als etwa 50 Mikroampere (0,05 Milliampere) und damit im physiologischen Bereich gehalten werden, ohne seine diagnostische und/oder therapeutische Wirksamkeit zu verlieren. Durch die Nähe, die die beiden Pole im Inneren des Elektrodenendgerätes zueinander haben, fließt der größte Strom im Bereich der Hand selbst. Der Strom, der durch den Körper fließt, liegt bei nur etwa 2 bis 4 Mikroampere je Arm. Der Querstrom durch den Körper, der sich durch Unsymmetrie ergeben könnte, ist nochmals ein Bruchteil davon. Fig. 1 zeigt einen Vergleich von Maximalmomentan -bzw. Spitzenwerten je Impuls-Nadel (Kurvenform = weißer, oberer Nadelimpuls, siehe Oszillogramm), aufgenommen mit einem erfindungsgemäßen zweipoligen Elektrodenendgerät. Der Impuls (weißer, oberer Trace) entspricht dem Strom, der in der Sensorfolie der Elektrode gemessen wird, und damit dem Strom, der zum Teil durch die Hand des Patienten, zum Teil durch die Umgebung "fließt" oder besser influenziert wird. Die andere (graue, untere) Kurve stellt den Verlauf des Wechselfeldes (der angelegten Wechselspannung) in der Senderfolie bzw. Treiberfolie der Elektrode dar, also die Ursache für den Influenzvorgang. Die Gesamtdauer des Impulses beträgt dabei ungefähr 200 bis 300 Nanosekunden.

Somit ergibt sich eine Ladung von etwa 4•10⁻⁶ Ampere • 200•10⁻¹² Sekunden oder 8•10⁻¹⁶ Amperesekunden je Nadelimpuls. Bei etwa 1 kHz "Nadelfrequenz" (statistische Wiederholrate) liefert dies im Mittel einen Gesamtstrom von 8•10⁻¹³ Ampere oder 0,8 Picoampere als vergleichbaren Dauerstrom durch einen Arm.

Werden erfindungsgemäß Elektrodenendgeräte verwendet, bei denen das elektrisch leitende Elektrodenmaterial des Elektrodenendgerätes wenigstens teilweise mit einem elektrisch nichtleitenden Überzug versehen ist und zwei voneinander elektrisch isolierte Elektroden (Pole) enthält, zwischen denen eine regelbare Wechselspannung angelegt werden kann, so wird ein unmittelbarer Kontakt zwischen der Haut und den elektrisch leitenden Oberflächen vermieden. Eine Übertragung der therapeutischen Funktion der Ströme und Wechselspannungen findet schonend auf kapazitivem Wege statt. Statt direkter Ströme von metallischen Oberflächen ist somit lediglich ein Influenz-Effekt des Erregerfeldes gegeben, der nur sehr geringe Körperströme verursacht.

Besonders bevorzugt ist ein erfindungsgemäßes Elektrodenendgerät, bei dem der Überzug mindestens die Hälfte, besser drei Viertel und am besten den gesamten für den Kontakt mit der Haut des Patienten vorgesehenen Bereich umfasst. Hierdurch wird der unmittelbare Kontakt des elektrisch leitenden metallischen Materials auch bei einem versehentlichen Verrutschen der Hand zuverlässig vermieden.

Der Überzug sollte eine Dicke von 0,005 bis 3 cm, bevorzugt im Bereich von 0,01 bis 1 cm, insbesondere 0,2 bis 0,5 cm aufweisen. Als ideal hat sich beispielsweise eine Dicke (Wandstärke) von 2 mm erwiesen, da auf diese Weise mechanische Stabilität, angenehmer Griff und zuverlässige Signalübertragung bei Eindämmung von potentiell schädigenden Überströmen zugleich in optimaler Weise gewährleistet werden kann.

Vorteilhafterweise enthält erfindungsgemäße Überzug, bzw. vorzugsweise besteht er aus Glas und/oder Kunststoff. Als Kunststoff hat sich insbesondere Acrylglas als vorteilhaft erwiesen. Grundsätzlich sieht die Erfindung jedoch auch die Verwendung anderer, durchsichtiger oder undurchsichtiger Materialien vor, solange die Materialien den mechanischen und chemischen Anforderungen an die Stabilität, Hitzeunempfindlichkeit, Schweißunempfindlichkeit, Desinfizierbarkeit von im Medizinbetrieb zu verwendenden Gegenständen genügt und im wesentlichen Nichtleitend sind, das heißt einen spezifischen Widerstand von mindestens 10¹⁰, besser mindestens 10¹² Ohmmeter aufweisen.

Das zweipolige Elektrodenendgerät der vorliegenden Erfindung weist bevorzugt einen isolierenden Abstand von 0,3 bis 2 cm, insbesondere von 0,8 bis 1,5 cm zwischen den beiden Polen (Elektroden) auf. Ein zu großer Abstand würde dazu führen, dass die therapeutischen Ströme zu gering würden bzw. ein gleichzeitiger Kontakt der Haut einer einzigen Hand mit beiden Polen erschwert oder verunmöglicht würde. Ein zu geringer Abstand birgt die Gefahr von Kurzschlüssen oder unbeabsichtigten Entladungen und damit Verlust der Sensibilität.

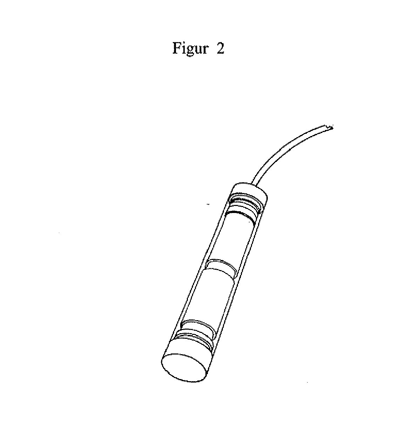

Für die äußere Form des Elektrodenendgeräts der Erfindung bestehen keine besonderen Einschränkungen. Es hat sich jedoch als besonders vorteilhaft herausgestellt, die Elektrodenendgeräte stabförmig auszubilden, damit sie gut in jeweils einer Hand gehalten werden können. Die Erfindung betrifft jedoch gleichermaßen als Platten, flexible Flächengebilde wie Textilien oder Kugeln ausgebildete Elektrodenendgeräte. Figur 2 zeigt eine bevorzugte stabförmige Ausführungsform der Erfindung.

In einer weiteren Ausführungsform betrifft die Erfindung ein Gerät zur Diagnose und/oder Therapie nach dem Prinzip des biofunktionellen Ansatzes umfassend mindestens ein erfindungsgemäßes Elektrodenendgerät. Besonders bevorzugt weist das Gerät zur Diagnose und/oder Therapie nach dem Prinzip des biofunktionellen Ansatzes mindestens zwei, bevorzugt genau zwei erfindungsgemäße Elektrodenendgeräte auf, die bevorzugt über flexible Kabel unlösbar oder lösbar, beispielsweise über elektrische Steckverbindungen mit dem Zentralgerät verbunden sind.

Das erfindungsgemäße Gerät zur Diagnose und/oder Therapie nach dem Prinzip des biofunktionellen Ansatzes ist besonders bevorzugt für physiologisch erträgliche Stromstärken zwischen den Elektroden eines Elektrodenendgerätes und den Elektroden mehrerer, insbesondere zweier Elektrodenendgeräte ausgelegt. Insbesondere sollten die Ströme zwischen den Elektroden einen Wert von 150, besser 100, am besten 50 oder ideal 40 Mikroampere nicht übersteigen.

## Patentansprüche

1. Einstückiges Elektrodenendgerät für Diagnose und/oder Therapie nach dem Prinzip des biofunktionellen Ansatzes, **dadurch gekennzeichnet, dass** es zwei voneinander elektrisch isolierte Elektroden enthält, zwischen denen eine Wechselspannung angelegt werden kann.

2. Einstückiges Elektrodenendgerät für Diagnose und/oder Therapie nach dem Prinzip des biofunktionellen Ansatzes, **dadurch gekennzeichnet, dass** das elektrisch leitende Elektrodenmaterial des Elektrodenendgerätes wenigstens teilweise mit einem elektrisch nichtleitenden Überzug versehen ist.

3. Einstückiges Elektrodenendgerät nach Anspruch 1, **dadurch gekennzeichnet, dass** das elektrisch leitende Elektrodenmaterial des Elektrodenendgerätes wenigstens teilweise mit einem elektrisch nichtleitenden Überzug versehen ist.

4. Elektrodenendgerät nach Anspruch 2 oder 3, **dadurch gekennzeichnet, dass** der Überzug den gesamten für den Kontakt mit der Haut des Patienten vorgesehenen Bereich umfasst.

5. Elektrodenendgerät nach einem der Ansprüche 2 bis 4, **dadurch gekennzeichnet, dass** der Überzug eine Dicke von 0,005 bis 3 cm, bevorzugt im Bereich von 0,01 bis 1 cm, insbesondere 0,2 bis 0,5 cm aufweist.

6. Elektrodenendgerät nach einem der Ansprüche 2 bis 5, **dadurch gekennzeichnet, dass** der Überzug Glas und/oder Kunststoff enthält oder aus Glas oder Kunststoff besteht, wobei der Kunststoff insbesondere Acrylglas ist.

7. Elektrodenendgerät nach einem der Ansprüche 1, 3 bis 6, **dadurch gekennzeichnet, dass** die Pole einen Abstand von 0,3 bis 2 cm, insbesondere von 0,8 bis 1,5 cm voneinander aufweisen.

8. Elektrodenendgerät nach einem der Ansprüche 1, 3 bis 7, **dadurch gekennzeichnet, dass** es stabförmig ausgebildet ist.

9. Gerät zur Diagnose und/oder Therapie nach dem Prinzip des biofunktionellen Ansatzes umfassend mindestens ein Elektrodenendgerät nach einem der Ansprüche 1 bis 8.

10. Gerät nach Anspruch 9, **dadurch gekennzeichnet, dass** es mindestens zwei, bevorzugt genau zwei Elektrodenendgeräte nach einem der Ansprüche 1 bis 7 umfasst.

11. Gerät nach Anspruch 10 oder 9, **dadurch gekennzeichnet, dass** es für physiologisch erträglichen Stromstärken zwischen den Elektroden eines Elektrodenendgerätes und den Elektroden mehrerer, insbesondere zweier Elektrodenendgeräte ausgelegt ist.

12. Gerät nach Anspruch 11, **dadurch gekennzeichnet, dass** die Ströme zwischen den Elektroden einen Wert von 150 Mikroampere nicht übersteigen.
